# FASCICULE DE BREVET EUROPEEN

(11) **EP 0 819 671 B1**
(45) Date de publication et mention de la délivrance du brevet: **31.10.2001**
(21) Numéro de dépôt: 97401496.1
(22) Date de dépôt: 26.06.1997
(51) Int. Cl.: C07C 219/08

(54) **Solutions aqueuses stabilisées de sels d'ammoniums quaternaires insaturés**
Stabilisierte wässrige Lösungen von ungesättigten, quaternären Ammoniumsalzen
Stabilised aqueous solutions of unsaturated quaternary ammonium salts

(30) Priorité: 08.07.1996 FR 9608476
(43) Date de publication de la demande: 21.01.1998
(73) Titulaire: Atofina, 92800 Puteaux (FR)
(72) Inventeur: Riondel, M. Alain, 57600 Forbach (FR); Legros, M. Robert, 60940 Monceaux (FR)
(74) Mandataire: Rieux, Michel

(56) Documents cités:
- EP-A- 0 250 325
- EP-A- 0 273 415
- EP-A- 0 302 122
- EP-A- 0 818 437
- FR-A- 2 642 424
- CHEMICAL ABSTRACTS, vol. 97, no. 20, 15 Novembre 1982 Columbus, Ohio, US; abstract no. 163659, SANYO CHEMICAL INDUSTRIES, LTD., JAPAN: "Stabilizing unsaturated ammonium salts" XP002025773 & JP 57 109 749 A (SANYO CHEMICAL INDUSTRIES, LTD., JAPAN)
- DATABASE WPI Section Ch, Week 9349 Derwent Publications Ltd., London, GB; Class A41, AN 93-392677 XP002025775 & JP 05 295 011 A (KURARAY CO LTD) , 9 Novembre 1993
- CHEMICAL ABSTRACTS, vol. 78, no. 4, 29 Janvier 1973 Columbus, Ohio, US; abstract no. 17513, NEGISHI, SUTEMI ET AL.: "Solution polymerization of acrylonitrile" page 79; XP002025774 & JP 47 037 712 B (TEIJIN)

## Description

La présente invention concerne des solutions aqueuses de sels d'ammoniums quaternaires insaturés (ci-après dénommés sels quaternaires), répondant à la formule (I) suivante : 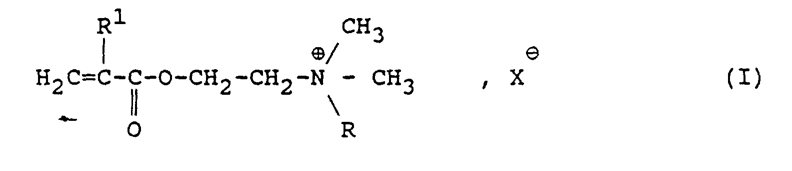 dans laquelle :
- R¹ représente un atome d'hydrogène ou un radical méthyle ;
- R représente un radical méthyle ou un radical benzyle ;
- X est choisi parmi Cl, Br, I ou -CH₃-SO₄,
les sels quaternaires (I) ayant été obtenus par réaction, en présence d'eau, du (méth)acrylate de N,N-diméthylaminoéthyle de formule (II) : 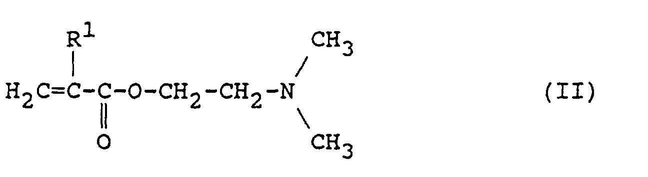 dans laquelle R¹ est tel que défini ci-dessus,
avec un agent quaternisant de formule (III) :

R-X (III)

dans laquelle R et X sont également tels que définis ci-dessus.

On utilise des solutions aqueuses de sels quaternaires (I) pour préparer des polymères destinés à servir de floculants dans le traitement des eaux.

Les principaux problèmes qui se posent lors de la synthèse des sels quaternaires (I) sont, d'une part, la sensibilité à l'eau des monomères (II), et, d'autre part, les risques de polymérisation du milieu réactionnel dans le réacteur et ceux des sels quaternaires (I) au stockage.

Les demandes de brevets japonais JP-A-57 109 747 et JP-A-57 109 749 décrivent un procédé pour stabiliser des sels de formule (I). Ce procédé consiste à ajouter à ces sels, au moins un agent chélatant qui peut être un acide amino carboxylique et respectivement un composé nitroso ou un composé de type quinone. En pratique, comme acide amino carboxylique, l'acide éthylène diamine tétra acétique (EDTA) et le sel trisodique de l'acide nitrilo triacétique ont été expérimentés, soit avec un composé nitroso, soit avec la benzoquinone pour stabiliser une solution aqueuse de chlorure de méthacryloyloxyéthyltriméthyl ammonium.

L'EDTA est également le seul agent séquestrant cité dans la demande de brevet européen EP-A-302 122 qui décrit un procédé de préparation de sels d'ammonium quaternaire insaturés par quaternisation dans un milieu constitué par de l'eau et un solvant organique aprotique.

La Société déposante a maintenant découvert que l'utilisation d'agents séquestrants particuliers, appartenant à la famille des acides amino carboxyliques et leurs sels, permet d'obtenir des solutions de sels possédant une excellente stabilité à la température ambiante.

La présente invention a donc d'abord pour objet une solution aqueuse stabilisée d'au moins un sel d'ammonium quaternaire insaturé, répondant à la formule (I) telle que définie ci-dessus, et ayant été obtenu par la réaction d'un monomère (II) et d'un agent quaternisant (III), comme indiqué ci-dessus, ladite solution aqueuse contenant au moins un stabilisant associé au monomère (II) et contenant en outre au moins un agent séquestrant pour métaux, caractérisée par le fait que l'agent séquestrant pour métaux est choisi parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique.

La teneur en agent(s) séquestrant(s) selon l'invention est, d'une manière générale, d'environ 1 à 100 ppm par rapport à la solution aqueuse de sel quaternaire de formule (I). Les agents séquestrants sont ajoutés préférentiellement dans le monomère (II) en début de réaction.

Les stabilisants sont choisis notamment parmi le 3,5-di-tert.-butyl-4-hydroxy-toluène, l'éther méthylique de l'hydroquinone, l'hydroquinone, le catéchol, le tert.-butyl-catéchol et leurs mélanges.

La teneur en agent(s) stabilisant(s) est, conformément à la présente invention, d'environ 20 à 1200 ppm par rapport à la solution aqueuse de sel quaternaire de formule (I).

Les solutions aqueuses de sels quaternaires selon l'invention sont des solutions à des concentrations notamment d'environ 50 à 85% en poids de sels quaternaires (I) dans l'eau.

La présente invention a également pour objet un procédé de fabrication d'une solution aqueuse stabilisée de sel d'ammonium quaternaire insaturé, répondant à la formule (I) telle que définie ci-dessus, à partir d'au moins un monomère (méth)acrylique et d'au moins un agent quaternisant des formules respectivement (II) et (III), également telles que définies ci-dessus, en présence d'au moins un stabilisant associé au monomère (II) introduit au début de la réaction, caractérisé par le fait qu'on ajoute au monomère (II) au début de la réaction et/ou à la solution aqueuse obtenue au moins un agent séquestrant pour métaux choisi parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique.

On a indiqué ci-dessus des exemples de stabilisants, et les quantités préférées de ceux-ci, ainsi que celles des agents séquestrants.

D'une manière générale, les agents séquestrants selon l'invention sont ajoutés sous la forme d'une solution aqueuse, car ils sont généralement disponibles sous cette forme. Ainsi, le sel pentasodique de l'acide diéthylène triamine penta acétique commercialisé sous la dénomination VERSENEX 80 se présente sous la forme d'une solution aqueuse à 40% en poids environ.

Une mise en oeuvre particulièrement préférée du procédé selon l'invention est celle qui fait l'objet du brevet européen EP-B-0 250 325. A cet effet :
(a) on fait réagir, dans un réacteur fermé, le monomère (méth)acrylique (II) avec 5 à 20% de la quantité pondérale nécessaire à la réaction, d'agent quaternisant (III), ce dernier étant introduit en continu dans le réacteur ; ou
(a') on introduit, dans un réacteur fermé, le monomère (méth)acrylique (II) et 5 à 20% en poids d'une solution aqueuse de sel quaternaire (I) par rapport au poids des monomères (méth)acryliques (II) comprenant de 50 à 85% en poids de sel quaternaire ;
(b) ensuite, on ajoute en continu l'eau et le reste d'agent quaternisant (III) jusqu'à l'obtention de la concentration souhaitée de sel d'ammonium quaternaire (I) dans l'eau ;
(c) on maintient la température pendant les étapes (a) ou (a') et (b) à une valeur comprise entre 30 et 60°C ; et
(d) pendant les étapes (a) ou (a') et (b) et à l'approche de la fin de la réaction en particulier, on maintient dans le milieu réactionnel un courant de gaz oxygéné, tel que le rapport en volume de gaz total à la sortie d'un réacteur sur l'oxygène introduit à l'entrée de ce même réacteur soit inférieur à 100/1.

De préférence, on impose, lors de la réaction, un rapport en volume de gaz sortant du réacteur sur l'oxygène introduit à l'entrée de ce réacteur, qui est inférieur à 50/1.

Dans le but d'éviter la sursaturation du milieu réactionnel en sel quaternaire (I), on impose pendant l'étape (b) un rapport molaire d'introduction d'eau sur l'agent quaternisant (III) qui est compris entre 2,2 et 3,7.

De préférence, on maintient, pendant les étapes (a) ou (a') et (b), une température comprise entre 45°C et 55°C.

De préférence encore, pendant l'étape (a) ou (a'), on introduit environ 10% en poids respectivement d'agent quaternisant (III) ou d'une solution aqueuse de sel quaternaire (I) comprenant 50 à 85% en poids de sel quaternaire.

Le procédé selon l'invention est, de préférence, conduit à une pression comprise entre la pression atmosphérique et 1,6 bar en pression absolue.

Lorsque l'agent quaternisant (III) est un composé volatil, gazeux à la température de réaction, l'introduction de l'agent quaternisant en cours de réaction est conduite de manière à limiter ses pertes dans les évents gazeux. Les pertes sont ainsi maintenues inférieures à 10% (molaire) de la stoechiométrie.

Le gaz en sortie du réacteur est ensuite conduit vers un dispositif de traitement visant à le débarrasser des traces de l'agent quaternisant (III) qu'il contient, en particulier dans le cas où ce dernier possède une tension de vapeur élevée ou est volatil à la température de réaction. De préférence, on envoie le gaz dans un second réacteur comprenant du monomère (méth)acrylique (II) dans lequel l'agent quaternisant (III) est piégé.

En fin de réaction, les traces d'agent quaternisant (III) dissous dans le mélange réactionnel sont éliminées par un balayage de ce dernier avec un fort débit de gaz oxygéné, par exemple de l'air.

Les agents quaternisants (III) convenant bien à la présente invention sont notamment les hydrocarbures halogénés, tels que le chlorure de méthyle, le bromure de méthyle, l'iodure de méthyle, le chlorure de benzyle, le bromure de benzyle et l'iodure de benzyle, ainsi que le sulfate de diméthyle.

Les exemples qui vont suivre, donnés à titre indicatif, permettent de mieux comprendre l'invention. Dans ces exemples, les pourcentages indiqués sont des pourcentages en poids, et les abréviations suivantes ont été utilisées :
- ADAME : acrylate de N,N-diméthylaminoéthyle
- MADAME : méthacrylate de N,N-diméthylaminoéthyle
- ADAMQUAT MC 80 : solution aqueuse de chlorure d'acryloyloxyéthyltriméthylammonium à 80%
- MADQUAT MC 75 : solution aqueuse de chlorure de méthacryloyloxyéthyltriméthylammonium à 75%
- EMHQ : éther méthylique de l'hydroquinone
- VERSENEX 80 : sel pentasodique de l'acide diéthylène triamine penta acétique (solution aqueuse à 40% en poids en matière active).

### Exemple 1 : Préparation d'ADAMQUAT MC 80

Dans un réacteur à double enveloppe, sous agitation, on charge 429 g d'ADAME stabilisé avec 800 ppm d'EMHQ.

Pendant toute la durée de la réaction, soit au total 6 heures, on maintient la température à 47°C, la pression atmosphérique et un débit d'air continu de 0,4 Nl/h à l'entrée du réacteur.

On introduit 20 g de chlorure de méthyle à un débit de 39 g/h pendant 0,5 heure, soit 12% de la quantité totale de CH₃Cl nécessaire à la réaction, puis on introduit simultanément 143 g d'eau et 139 g de chlorure de méthyle à des débits respectifs de 32 g/h et 39 g/h, soit un rapport molaire eau/CH₃Cl égal à 2,3.

Ensuite, on injecte dans le produit fabriqué de l'air à raison de 5 Nl/h pendant 0,5 heure à chaud. On récupère 720 g d'ADAMQUAT MC 80.

Le produit ainsi obtenu est stabilisé avec 10 ppm de VERSENEX 80.

Dans le Tableau 1 suivant, on a fait figurer le résultat d'un essai de stabilité au stockage de la solution aqueuse de sel obtenue. Pour effectuer cet essai de stabilité, on immerge, dans un bain d'huile thermostaté (température-: 92°C), un tube à essai fermé, rempli à 80% avec l'ADAMQUAT MC 80 dopé avec le VERSENEX 80, et on apprécie l'efficacité de ce dernier en mesurant la durée avant polymérisation de l'ADAMQUAT MC 80.

### Exemple 2

On procède comme à l'Exemple 1, en remplaçant le Versenex 80 respectivement par un autre agent séquestrant comme indiqué dans le Tableau 1.

Les résultats sont également rapportés dans le Tableau 1, en même temps que ceux d'un exemple témoin, sans séquestrant (Exemple 3) et d'exemples comparatifs 4 à 7 conduits avec des agents séquestrants n'appartenant pas à l'invention.

**TABLEAU 1**

| Exemple | Agent séquestrant | Durée avant polymérisation (h) |
|---|---|---|
| 1 | Versenex 80 | 280 |
| 2 | Sel trisodique de l'acide N-hydroxyéthyl éthylène diamine triacétique | 300 |
| 3 (témoin) | Sans agent séquestrant | 3,25 |
| 4 (comparatif) | Poly(acide acrylique) | 1 |
| 5 (comparatif) | Poly(acrylate de sodium) | 2 |
| 6 (comparatif) | Sel tétrasodique de l'acide éthylène diamine tétraacétique | 72 |
| 7 (comparatif) | Acide éthylène diamine tétra acétique | 154 |

## Revendications

1. Solution aqueuse stabilisée de sel(s) d'ammonium quaternaire insaturé(s), répondant à la suivante : 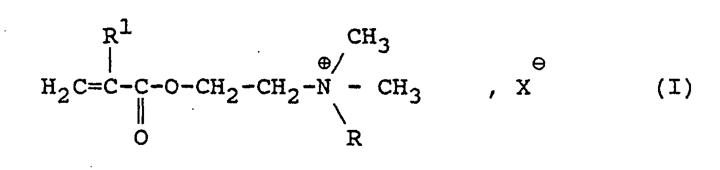 dans laquelle :
- R¹ représente un atome d'hydrogène ou un radical méthyle ;
- R représente un radical méthyle ou un radical benzyle ;
- X est choisi parmi Cl, Br, I ou -CH₃-SO₄,
les sels quaternaires (I) ayant été obtenus par réaction, en présence d'eau, du (méth)acrylate de N,N-diméthylaminoéthyle de formule (II) : 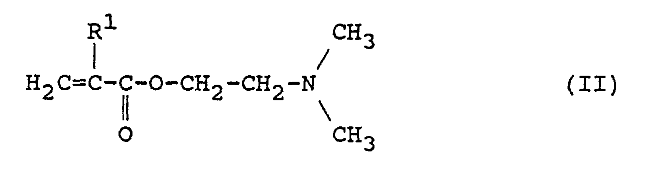 dans laquelle R¹ est tel que défini ci-dessus,
avec un agent quaternisant de formule (III) :
R-X (III)
dans laquelle R et X sont également tels que définis ci-dessus ;
ladite solution aqueuse contenant au moins un stabilisant associé au monomère (II) et contenant en outre au moins un agent séquestrant pour métaux, **caractérisée par le fait que** l'agent séquestrant pour métaux est choisi parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique sauf pour les composés de formule II et III dans lesquels R et R₁ représentent un radical méthyle et pour lesquels l'agent séquestrant est le sel pentasodique de l'acide diéthylène triamine penta acétone.

2. Solution aqueuse selon la revendication 1, **caractérisée par le fait que** la teneur en agent(s) séquestrant(s) est de 1 à 100 ppm par rapport à la solution aqueuse de sel quaternaire (I).

3. Solution aqueuse selon l'une des revendications 1 et 2, **caractérisée par le fait que** les stabilisants sont choisis par le 3,5-diterbutyl-4-hydroxytoluène, l'éther méthylique d'hydroquinone, l'hydroquinone, le catéchol, le tert.-butyl catéchol et les mélanges de ces stabilisants.

4. Solution aqueuse selon l'une des revendications 1 à 3, **caractérisée par le fait que** la teneur en agent(s) stabilisant(s) est de 20 à 1200 ppm par rapport à la solution aqueuse de sel quaternaire (I).

5. Solution aqueuse selon l'une des revendications 1 à 4, **caractérisée par le fait que** sa concentration en sel quaternaire (I) est de 50 à 85% en poids.

6. Procédé de fabrication d'une solution aqueuse stabilisée de sel d'ammonium quaternaire insaturé, telle que définie à la revendication 1, à partir d'au moins un monomère (méth)acrylique et d'au moins un agent quaternisant des formules respectivement (II) et (III), également telles que définies à la revendication 1, en présence d'au moins un stabilisant associé au monomère (II) introduit au début de la réaction, **caractérisé par le fait qu'**on ajoute au monomère (II) au début de la réaction et/ou à la solution aqueuse obtenue au moins un agent séquestrant pour métaux choisi parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique.

7. Procédé selon la revendication 6, **caractérisé par le fait que** le ou les agents séquestrants sont ajoutés sous la forme d'une solution aqueuse.

8. Procédé selon l'une des revendications 6 et 7, **caractérisé par le fait que** :
(a) on fait réagir, dans un réacteur fermé, le monomère (méth)acrylique (II) avec 5 à 20% de la quantité pondérale nécessaire à la réaction, d'agent quaternisant (III), ce dernier étant introduit en continu dans le réacteur ; ou
(a') on introduit, dans un réacteur fermé, le monomère (méth)acrylique (II) et 5 à 20% en poids d'une solution aqueuse de sel quaternaire (I) par rapport au poids des monomères (méth)acryliques (II) comprenant de 50 à 85% en poids de sel quaternaire ;
(b) ensuite, on ajoute en continu l'eau et le reste d'agent quaternisant (III) jusqu'à l'obtention de la concentration souhaitée de sel d'ammonium quaternaire (I) dans l'eau ; et
(c) on maintient la température pendant les étapes (a) ou (a') et (b) à une valeur comprise entre 30 et 60°C ; et
(d) pendant les étapes (a) ou (a') et (b) et à l'approche de la fin de la réaction en particulier, on maintient dans le milieu réactionnel un courant de gaz oxygéné, tel que le rapport en volume de gaz total à la sortie d'un réacteur sur l'oxygène introduit à l'entrée de ce même réacteur soit inférieur à 100/1.

9. Procédé selon la revendication 8, **caractérisé par le fait que** l'on conduit la réaction à une pression absolue comprise entre la pression atmosphérique et 1,6 bar.

10. Procédé selon l'une des revendications 8 et 9, **caractérisé par le fait qu'**on conduit le gaz sortant du réacteur vers un dispositif de traitement visant à le débarrasser des traces d'agent quaternisant R-X, dispositif qui est constitué d'un second réacteur de quaternisation comprenant du monomère (méth)acrylique.

## Patentansprüche

1. Wäßrige Lösung eines oder mehrerer ungesättigter quartärer Ammoniumsalzes der folgenden Formel (I) 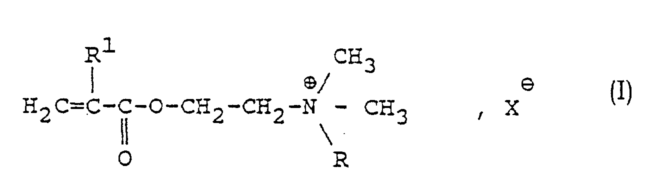 in der bedeuten:
- R¹ Wasserstoff oder Methyl,
- R Methyl oder Benzyl,
- X ein Anion, das unter Cl, Br, I und CH₃SO₄ ausgewählt ist wobei die quartären Salze der Formel (I) durch Umsetzung des N,N-Dimethylaminoethyl(meth)acrylats der Formel (II): 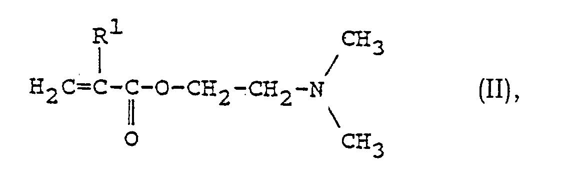
in der R¹ wie weiter oben definiert ist, mit einem Quaternisierungsmittel der Formel (III)
R-X (III)
in Gegenwart von Wasser erhalten werden, in der R und X ebenfalls wie weiter oben definiert sind,
wobei die wäßrige Lösung mindestens ein Stabilisierungsmittel enthält, das mit dem Monomer (II) kombiniert ist, und außerdem mindestens ein Maskierungsmittel für Metalle enthält, **dadurch gekennzeichnet, daß** das Maskierungsmittel für Metalle unter Diethylentriaminpentaessigsäure, dem Pentanatriumsalz der Diethylentriaminpentaessigsäure, N-Hydroxyethylethylendiamintriessigsäure und dem Trinatriumsalz der N-Hydroxyethylethylendiamintriessigsäure ausgewählt ist, mit Ausnahme der Verbindungen der Formeln II und III, in denen R und R¹ Methyl bedeutet, für die das Maskierungsmittel aus dem Pentanatriumsalz der Diethylentriaminpentaessigsäure besteht.

2. Wäßrige Lösung nach Anspruch 1, **dadurch gekennzeichnet, daß** ihr Gehalt an dem oder den Maskierungsmitteln im Bereich von 1 bis 100 ppm, bezogen auf die wäßrige Lösung des quartären Salzes (I), liegt.

3. Wäßrige Lösung nach einem der Ansprüche 1 und 2, **dadurch gekennzeichnet, daß** die Stabilisierungsmitteln unter 3,5-Di-*tert*.-butyl-4-hydroxytoluol, dem Hydrochinonmethylether, Hydrochinon, Brenzcatechin, tert.-Butylbrenzcatechin und den Gemischen dieser Verbindungen ausgewählt sind.

4. Wäßrige Lösung nach einem der Ansprüche 1 bis 3, **dadurch gekennzeichnet, daß** der Gehalt an dem oder den Stabilisierungsmittel(n) im Bereich von 20 bis 1200 ppm, bezogen auf die wäßrige Lösung des quartären Salzes (I), liegt.

5. Wäßrige Lösung nach einem der Ansprüche 1 bis 4, **dadurch gekennzeichnet, daß** ihre Konzentration an quartärem Salz (I) im Bereich von 50 bis 85 Gew.-% liegt.

6. Verfahren zur Herstellung einer stabilisierten wäßrigen Lösung eines ungesättigten quartären Ammoniumsalzes, das wie in Anspruch 1 definiert ist, aus mindestens einem (Meth)acrylmonomer und mindestens einem Quaternisierungsmittel der Formel (II) bzw. (III), das ebenfalls wie in Anspruch 1 definiert ist, in Gegenwart von mindestens einem Stabilisierungsmittel, das mit dem Monomer (II) kombiniert ist und das zu Beginn der Umsetzung zugegeben wird, **dadurch gekennzeichnet, daß** zu Beginn der Umsetzung zu dem Monomer (II) und/oder zu der erhaltenen wäßrigen Lösung mindestens ein Maskierungsmittel für Metalle gegeben wird, das unter Diethylentriaminpentaessigsäure, dem Pentantriumsalz der Diethylentriaminpentaessigsäure, N-Hydroxyethylethylendiamintriessigsäure und dem Trinatriumsalz der N-Hydroxyethylethylendiamintriessigsäure ausgewählt ist, mit Ausnahme der Verbindungen der Formeln II und III, in denen R und R¹ Methyl bedeutet, für die das Maskierungsmittel aus dem Pentanatriumsalz der Diethylentriaminpentaessigsäure besteht.

7. Verfahren nach Anspruch 6, **dadurch gekennzeichnet, daß** das oder die Maskierungsmittel in Form einer wäßrigen Lösung zugegeben werden.

8. Verfahren nach einem der Ansprüche 6 und 7, **dadurch gekennzeichnet, daß**
(a) das (Meth)acrylmonomer (II) in einem geschlossenen Reaktor mit 5 bis 20 % der für die Umsetzung erforderlichen Gewichtsmenge eines Quaternisierungsmittels (III) umgesetzt wird, wobei das Quaternisierungsmittel kontinuierlich in den Reaktor eingebracht wird, oder
(a') das (Meth)acrylmonomer (II) und 5 bis 20 Gew.-% einer wäßrigen Lösung des quartären Salzes, bezogen auf das Gewicht der (Meth)acrylmonomere (II), die 50 bis 85 Gew.-% quartäres Salz enthält, in einen geschlossenen Reaktor gegeben werden,
(b) anschließend kontinuierlich Wasser und das restliche Quaternisierungsmittel (III) zugegeben werden, bis die gewünschte Konzentration an quartärem Ammoniumsalz (I) in Wasser erhalten wird,
(c) während der Schritte (a) bzw. (a') und (b) eine Temperatur von 30 bis 60 °C beibehalten wird,
(d) während der Schritte (a) oder (a') und (b) und insbesondere wenn man sich dem Ende der Umsetzung nähert ein Strom eines sauerstoffhaltigen Gases in einer solchen Menge in das Reaktionsmedium eingeleitet wird, daß das Verhältnis des gesamten Gasvolumens am Auslaß des Reaktors zu dem am Einlaß des Reaktors zugegebenen Sauerstoff kleiner als 100/1 ist.

9. Verfahren nach Anspruch 8, **dadurch gekennzeichnet, daß** die Umsetzung bei einem absoluten Druck im Bereich vom Atmosphärendruck bis 1,6 bar durchgeführt wird.

10. Verfahren nach einem der Ansprüche 8 und 9, **dadurch gekennzeichnet, daß** das aus dem Reaktor austretende Gas in eine Vorrichtung geleitet wird, die dazu dient, Spuren des Quaternisierungsmittels R-X zu entfernen, wobei die Vorrichtung aus einem zweiten Quaternisierungsreaktor besteht, der (Meth)acrylmonomer enthält.

## Claims

1. Stabilized aqueous solution of unsaturated quaternary ammonium salt(s), corresponding to the following: 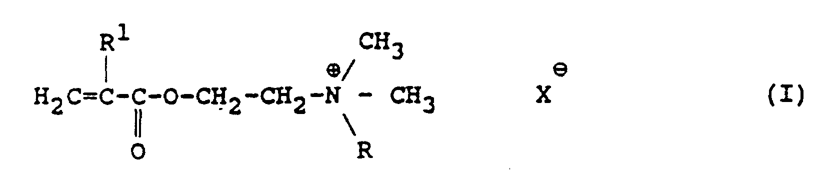 in which:
- R¹ represents a hydrogen atom or a methyl radical;
- R represents a methyl radical or a benzyl radical;
- X is chosen from Cl, Br, I or CH₃-SO₄,
the quaternary salts (I) having been obtained by reaction, in the presence of water, of N,N-dimethylaminoethyl (meth)acrylate of formula (II): 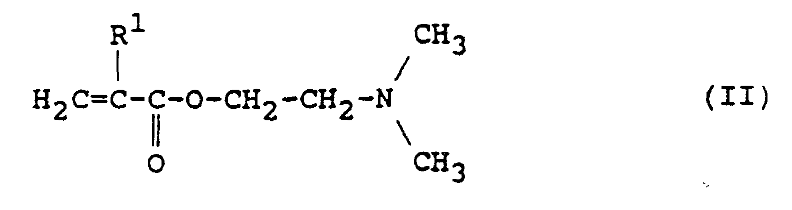 in which R¹ is as defined above,
with a quaternizing agent of formula (III):
R-X (III)
in which R and X are also as defined above;
the said aqueous solution containing at least one stabilizing agent in combination with the monomer (II) and additionally containing at least one sequestering agent for metals, **characterized in that** the sequestering agent for metals is chosen from diethylenetriaminepentaacetic acid, the pentasodium salt of diethylenetriaminepentaacetic acid, N-(hydroxyethyl)ethylene-diaminetriacetic acid and the trisodium salt of N-(hydroxyethyl)ethylenediaminetriacetic acid, except for the compounds of formulae II and III in which R and R₁ represent a methyl radical and for which the sequestering agent is the pentasodium salt of diethylenetriaminepentaacetic acid.

2. Aqueous solution according to Claim 1, **characterized in that** the content of sequestering agent(s) is from 1 to 100 ppm with respect to the aqueous solution of quaternary salt (I).

3. Aqueous solution according to one of Claims 1 and 2, **characterized in that** the stabilizing agents are chosen from 3,5-di-tert-butyl-4-hydroxytoluene, hydroquinone methyl ether, hydroquinone, catechol, tert-butylcatechol and the mixtures of these stabilizing agents.

4. Aqueous solution according to one of Claims 1 to 3, **characterized in that** the content of stabilizing agent(s) is from 20 to 1200 ppm with respect to the aqueous solution of quaternary salt (I).

5. Aqueous solution according to one of Claims 1 to 4, **characterized in that** its concentration of quaternary salt (I) is from 50 to 85% by weight.

6. Process for the manufacture of a stabilized aqueous solution of unsaturated quaternary ammonium salt, as defined in Claim 1, from at least one (meth)acrylic monomer and from at least one quaternizing agent of the formulae (II) and (III) respectively, also as defined in Claim 1, in the presence of at least one stabilizing agent in combination with the monomer (II) introduced at the beginning of the reaction, **characterized in that** at least one sequestering agent for metals is added to the monomer (II) at the beginning of the reaction and/or to the aqueous solution obtained, the sequestering agent for metals being chosen from diethylenetriaminepentaacetic acid, the pentasodium salt of diethylenetriaminepentaacetic acid, N-(hydroxyethyl)-ethylenediaminetriacetic acid and the trisodium salt of N-(hydroxyethyl)ethylenediaminetriacetic acid.

7. Process according to Claim 6, **characterized in that** the sequestering agent or agents are added in the form of an aqueous solution.

8. Process according to one of Claims 6 and 7, **characterized in that**:
(a) the (meth)acrylic monomer (II) is reacted, in a closed reactor, with 5 to 20% by weight of the amount of quaternizing agent (III) necessary for the reaction, this quaternizing agent being introduced continuously into the reactor; or
(a') the (meth)acrylic monomer (II) and 5 to 20% by weight of an aqueous solution of quaternary salt (I) with respect to the weight of the (meth)acrylic monomers (II) comprising from 50 to 85% by weight of quaternary salt are introduced into a closed reactor;
(b) water and the remainder of the quaternizing agent (III) are then continuously added until the desired concentration of quaternary ammonium salt (I) in the water is obtained;
(c) the temperature is maintained, during the stages (a) or (a') and (b), at a value of between 30 and 60°C; and
(d) during the stages (a) or (a') and (b) and at the approach of the end of the reaction in particular, a stream of oxygen-containing gas is maintained in the reaction mixture, such that the ratio by volume of total gas at the outlet of a reactor to the oxygen introduced at the inlet of this same reactor is less than 100/1.

9. Process according to Claim 8, **characterized in that** the reaction is carried out at an absolute pressure of between atmospheric pressure and 1.6 bar.

10. Process according to one of Claims 8 and 9, **characterized in that** the gas exiting from the reactor is conveyed to a treatment device which is targeted at ridding it of the traces of quaternizing agent R-X, which device is composed of a second quaternization reactor comprising (meth)acrylic monomer.
